Europäisches Patentamt

European Patent Office

Office européen des brevets

(11) Numéro de publication: **0 107 526**
**B1**

(12) # FASCICULE DE BREVET EUROPEEN

(45) Date de publication du fascicule du brevet:
**29.10.86**

(51) Int. Cl.⁴: **A 61 L 15/06,** A 61 L 15/03

(21) Numéro de dépôt: **83401748.5**

(22) Date de dépôt: **05.09.83**

(54) **Pâte pour la protection de la peau.**

(30) Priorité: **07.09.82 FR 8215191**

(43) Date de publication de la demande:
**02.05.84 Bulletin 84/18**

(45) Mention de la délivrance du brevet:
**29.10.86 Bulletin 86/44**

(84) Etats contractants désignés:
**AT BE CH DE GB IT LI LU NL SE**

(56) Documents cités:
**WO - A - 82/00005
WO - A - 82/00099
FR - A - 2 393 838
FR - A - 2 429 251**

(73) Titulaire: **LABORATOIRES BIOTROL S.A., 1, rue du Foin,
F-75140 Paris Cedex 03 (FR)**

(72) Inventeur: **Maupetit, Philippe, 23, rue François Massé,
F-93600 Aulnay sous Bois (FR)**
Inventeur: **Demoulin, Bernard, "Villa Tutina" Vieille
Route de Saint-Pée, F-64310 St Pee S/Nivelle (FR)**

(74) Mandataire: **Phélip, Bruno et al, c/o Cabinet Harlé &
Phélip 21, rue de la Rochefoucauld, F-75009 Paris (FR)**

## Description

La présente invention concerne des produits pour la protection de la peau, notamment contre les agressions des écoulements corporels des suintements de plaies (chirurgicales ou accidentelles) et/ou des matières s'écoulant des stomies urinaires ou digestives, et tout particulièrement leur application comme pâte pour former un joint entre les stomies et les appareillages classiquement utilisés.

Il est, en effet, important d'assurer une protection, soit de la peau susceptible de subir l'aggression des suintements des plaies chirurgicales ou accidentelles, soit autour d'une stomie, de la peau au contact de l'urine (pour une urétérostomie) ou des selles (pour une colostomie et une iléostomie). En ce qui concerne les stomies, elles ne sont, en effet, pas toutes d'une forme parfaitement régulière et elles présentent des anfractuosités, qui sont des zones où la peau n'est pas protégée par l'appareil de recueil qui y est normalement appliqué.

De plus, les appareillages de recueil mis à la disposition des stomisés présentent des diamètres d'ouverture standardisés, qui ne correspondent pas nécessairement à la taille réelle de la stomie à équiper.

Il existe déjà sur le marché des pâtes dont on prétend qu'elles peuvent servir de barrière aux matières agressives. Ces pâtes connues présentent cependant certains inconvénients ou occasionnent des difficultés à leurs utilisateurs.

C'est ainsi que les pâtes actuellement sur le marché comportent un alcool, une cétone ou un éther, comme liant principal et de ce fait même, doivent être contre-indiquées pour les peaux irritées ou sensibles.

Dans les brevets FR-A-78 16326 et FR-A-78 16615 sont décrites des compositions adhésives utilisées pour joints de stomies, à base de polymères naturels ou synthétiques mais se présentant sous forme de plaque et donc d'application localisée mal aisée.

On a maintenant trouvé et mis au point une pâte ayant une formulation originale, qui peut s'appliquer et s'enlever aisément et qui, une fois mise en place, protège toute la zone de peau entournant les plaies ou la stomie contre les acides et enzymes digestifs, les selles alcalines, l'urine et autres matières agressives. De plus, cette pâte s'est avérée compatible avec tous les pansements et tous les sytèmes de fixation de poches de recueil ou de drainage actuellement connus (poches adhésives, poches avec joint de Karaya, poches avec joint ou support synthétique).

La présente invention a pour premier objet une pâte de protection de la peau, caractérisée en ce qu'elle comprend fondamentalement:

a) au moins un hydrocolloïde,
b) de la carboxyméthylcellulose insolubilisée et
c) une polyvinylpyrrolidone (PUP) ayant une masse moléculaire de l'ordre de 25 000 à 30 000,

notamment en tant qu'épaississants,
d) de l'eau déminéralisée, en tant que liant, associée à
e) de l'huile et
f) un stéarate appropriés, ainsi que
g) de la polyvinylpyrrolodine réticulée et insolubilisée, et avantageusement
– du sorbitol et/ou du polyéthylène glycol. Selon une forme de réalisation préférée, les composants essentiels de la pâte sont dans les rapports et proportions ci-après, en poids/poids:

– poudre/eau 1 à 3,5 environ, de préférence 1,5 à 1,9;
– stéarate/(eau + huile + sorbitol et/ou polyethylene glycol) $\geq 0{,}10$, de préférence 0,15 environ;
– PVP/eau 0,5 à 2 environ;
et/ou un autre produit superabsorbent tiré d'hydro colloïdes et selon une forme de mise en œuvre également préférée:

– % de PVPP $\leq 3\%$ en poids/poids de la pâte finie.

Dans ces rapports, ainsi que dans la suite du présent texte, on entend par poudre l'ensemble des composants pulvérulents, mélangés entre eux, à savoir: l'hydrocolloïde, la carboxyméthylcellulose insolubilisée (désignée en abrégé par CMC), et la polyvinylpyrolidone (dénommée en abrégé PVP); d'autre part PVPP désigne la polyvinylpyrrolidone réticulée et insolubilisée.

Une pâte correspondant à une telle formulation s'est avérée exceptionnellement aisée à appliquer et à enlever et, une fois mise en place sur une zone de peau portant une plaie ou sur le pourtour d'une stomie, s'avère protéger avec une efficacité inconnue jusqu'alors, toute la zone de peau entourant la plaie ou la stomie contre les acides et enzymes digestifs, les selles alcalines, l'urine et les autres matières agressives habituelles.

De manière plus générale, il apparaît qu'une telle pâte peut, de façon inattendue, être utilisée aussi comme support et/ou véhicule neutre pour des compositions pharmaceutiques pour administration percutanée lente de divers principes actifs à diffusion transdermique (encore appelés produits «transdermiques»). De tels produits transdermiques sont actuellement connus et utilisés. Il est clair que, dans ce type d'application la pâte protège aussi bien la peau que le produit transdermique lui-même contre toute agression extérieure.

De plus, du fait que la base liante de la pâte est essentiellement de l'eau déminéralisée (alors que dans toutes les formulations actuellement commercialisées, il apparaît que la base liante principale est un alcool, une cétone ou un éther), la pâte selon l'invention n'est pas contre-indiquée pour les peaux irritées comme le sont les formulations de pâtes actuellement commercialisées; au contraire, la pâte selon l'invention comprend une association de base liante aqueuse avec de l'huile (telle qu'une huile de paraffine ou autres), formant une émulsion qui permet à un autre élément de la formulation, la polyvinylpyrrolidone réticulée et insolubilisée, de valoriser ses qualités filmogènes particulièrement efficaces pour la régénérescence des peaux abîmées.

Par ailleurs, la carboxyméthylcellulose insolubilisée (c'est-à-dire réticulée ou insolubilisée e toute autre manière selon des méthodes connues en soi) est apparue conférer à la pâte une fonction d'absorption exceptionnellement importante de l'humidité et des effluents aqueux sortant du corps du patient. Sous l'effet de l'humidité ainsi reçue, elle donne à la pâte une texture de gel ayant une viscosité élevée et lui confère la propriété de se transformer en barrière imperméable à toute phase liquide extérieure, quel qu'en soit le pH.

Outre les composants essentiels ou optionnels indiqués plus haut, la pâte selon l'invention peut également comprendre des composants secondaires choisis parmi les constituants et adjuvants classiques, et, à ce titre, notamment des conservateurs, tels que par exemple de l'acide sorbique ou du parahydroxybenzoate de méthyle sodé.

L'hydrocolloïde ou le mélange d'hydrocolloïdes que comprend la pâte selon l'invention peut être choisi à convenance par l'homme du métier parmi les hydrocolloïdes connus ou formulés à partir de plusieurs de ceux-ci. Ce peuvent être des hydrocolloïdes extraits de végétaux (graines ou algues) ou des hydrocolloïdes synthétisés par fermentation. Des exemples d'hydrocolloïdes sont le guar, la caroube, les alginates et les carraghénanes. On préfère les alginates, et tout particulièrement les alginates alcalins (notamment l'alginate de sodium), qui sont d'excellents épaississants, facilement solubles et inaptes à créer des zones de jonction dans le milieu à épaissir et plus généralement tout produit superabsorbant, tel que ceux conçus à partir d'hydrocolloïdes (par exemple le produit commercialisé sous la dénomination Cecalgum® par la Société Ceca et issu d'acide alginique) peut être utilisé dans le même but, avec les hydrocolloïdes ou à leur place.

La carboxyméthylcellulose insolubilisée peut être, par exemple, le produit commercialisé sous la dénomination Aqualon® par la Société Hercules France, qui est un composant super-absorbant.

La polyvinylpyrrolidone ayant une masse moléculaire de l'ordre de 25 000 à 30 000 est une poudre très fine qui, outre ses propriétés filmogènes inhibitrices bactériennes et adhésives, permet, en solution aqueuse, grâce à son très grand pouvoir liant, de conférer une bonne fluidité à la pâte. Toutes choses égales par ailleurs, la proportion de cette poudre de PVP par rapport à l'eau semble avoir une grande influence sur la viscosité de la pâte; pour qu'on obtienne une pâte ni trop fluide, ni trop épaisse, il convient de maintenir le rapport PVP/eau en pratique entre 0,5 et 2 environ, de préférence entre 0,6 et 1,25 environ.

L'huile et le stéarate appropriés, servant à fixer l'eau déminéralisée qui est le liant de la pâte peuvent être choisis respectivement parmi:

– l'huile paraffinique de qualité Codex (France), telle que par exemple une huile de Vaseline®, qui se présente sous forme liquide et se met en émulsion dans la pâte avec la phase aqueuse;

– un ester gras de l'acide stéarique, tel que par exemple, du palmitostéarate de glycol. En particulier, le palmitostéarate de glycol associé à un phosphate d'alcool gras polyoxyéthyléné, que se présente sous forme de cire, peut servir de base gélifiante anionique acide pour mélange eau-huile, c'est un autoémulsifiant. Mais on peut aussi utiliser d'autres stéarates d'alcools gras, tels que par exemple le palmitostéarate de glycérol et de polyoxyéthylène glycol, ou le palmitostéarate d'éthylène glycol et de polyéthylène glycol.

La polyvinylpyrrolidone réticulée et insolubilisée (PVPP) est une poudre très fine ayant une grande inertie chimique, une adhésivité en milieu humide, une grande hydrophilie, une insolubilité en milieux aqueux et de bonnes propriétés filmogènes.

La pâte selon l'invention peut, en outre, comprendre su sorbitol et/ou du polyéthylène glycol.

Le sorbitol se présente sous forme de poudre; il constitue un complément de liant en phase aqueuse et permet, si on le souhaite, de diminuer la quantité du liant qu'est l'eau. Dans la pratique courante, on l'utilise sous forme de solution aqueuse à 70% et les quantités indiquées plus loin pour le sorbitol correspondent à des quantités d'une telle solution de sorbitol (noté sorbitol aqueux).

Le polyéthylène glycol est un produit qui permet d'améliorer, si on le désire, la coulabilité de la pâte, tout en permettant de réduire la proportion d'huile paraffinique. Il se présente sous forme de poudre pour des masses moléculaires de 4000 à 6000 et sous forme liquide pour des masses moléculaires de l'ordre de 400.

Si on le désire, on peut utiliser l'un ou l'autre de ces constituants optionnels, ou les deux, en choissant pour chacun d'eux le grade le plus approprié.

Les proportions respectives des constituants susdits peuvent être choisies à convenance par l'homme du métier, qui peut recourir à des essais de routine pour ce faire, et, déterminer si certaines proportions posées au départ empiriquement peuvent effectivement convenir. A cet égard, il est préférable que soient respectés les rapports poudre/eau, stéarate/(eau + huile + sorbitol) et PVP/eau, ainsi que la proportion de PVPP, indiqués plus haut comme étant avantageux. Les quantités de chacun des composants individuels sont alors, soit libres, soit conditionnées par ces rapports et proportions.

En ce qui concerne l'addition éventuelle de conservateurs à cette pâte, on préfère le couple p-hydroybenzoate de méthyle sodé et acide sorbique, qui est un fongicide, de qualité Codex (France), permettant d'avoir une synergie à des dosages en poids respectivement de 1/1000 et 0,5/1000 par rapport au poids de la pâte. Ces conservateurs ne sont bien entendu cités qu'à titre d'exemple. Le choix des conservateurs n'est conditionné que par leur inscription sur Codex français et leur pouvoir inhibiteur intrinsèque lors de leur association avec les autres constituants de la pâte.

Le procédé de fabrication de la pâte selon l'invention fait appel à des techniques connues de l'homme du métier; il peut consister, par exemple, en l'une des deux méthodes opératoires suivantes;

I) a. On mélange de l'huile, le stéarate et de l'eau et on porte le mélange à environ 70°C, en agitant, afin de réaliser une émulsion.

b. On mélange la PVPP et le sorbitol ou le polyéthylène glycol ou les deux et, si on le désire, des conservateurs.

c. On ajoute à la PVP de l'eau déminéralisée, on agite jusqu'à une dissolution complète en maintenant le mélange à une température inférieure à 70°C; tout en agitant, on y ajoute l'hydrocolloïde et ensuite la CMC; une fois que la préparation ainsi réalisée est bien homogène, on maintient la température à moins de 70°C et on ajoute sous agitation les mélanges résultant des étapes a et b.

II) a. On porte le stéarate à 70°C et on le mélange à l'huile de paraffine.

b. D'autre part, on ajoute à la PVPP, si on le désire, le sorbitol et, éventuellement, des conservateurs.

c. On ajoute sous agitation de l'eau déminéralisée à la PVP; ensuite, on ajoute successivement et toujours sous agitation, l'hydrocolloïde et la CMC; on ajoute à cette préparation le mélange obtenu dans l'étape 2, en maintenant la température à moins de 70°C et on ajoute sous agitation, le produit de l'étape a.

Dans ces modes opératoires, on peut aussi prévoir l'addition de polyéthylène glycol, en remplacement d'une partie du sorbitol ou, à la place de celui-ci, en fonction de ce que l'on désire.

La présente invention a également pour objet l'application des pâtes susdites pour la protection de la peau, notamment contre les agressions des suintements de plaies et/ou des matières s'écoulant des stomies urinaires ou digestives. L'indice primaire d'irritation cutanée de la peau avec des pâtes selon l'invention a été mesuré par un procédé couramment utilisé et les essais ont montré que les pâtes n'avaient pas d'activité irritante. Elles ont été utilisées pour la protection des stomies de formes irrégulières, lorsqu'il existe des sillons et des fissures cutanés tels que les adhésifs des poches de recueil habituels ne peuvent adhérer correctement à la zone péristomiale, laissant donc le passage aux émissions corporelles agressives. On a constaté que les pâtes étaient parfaitement compatibles avec les adhésifs courants qui s'y collent parfaitement et que la durée de protection, qui est fonction de l'agressivité des émissions corporelles pouvait aller d'un jour à plus de huit jours, l'élimination du reste de pâte, avant son remplacement, pouvant se faire par simple lavage à l'eau savonneuse.

Un autre objet de l'invention est encore l'application des pâtes susdites comme support et/ou véhicule neutre pour des substances ou des préparations à activité thérapeutique à diffusion lente pour traitement percutané prolongé.

L'invention est décrite plus concrètement en réference aux exemples de réalisation ci-après, qui sont purement illustratifs et ne la limitent aucunement. Dans ces exemples, les quantités sont exprimées, sauf indication contraire, en parties en poids pour 100 parties de la pâte finie, exception faite du poids de conservateurs qu'elle pourrait également comprendre.

Exemple 1

a) on a ajouté, à 6 parties de palmitostéarate de glycérol et de polyoxyéthylène glycol, 3,3 parties d'huile de paraffine et 7 parties d'eau. On a porté le mélange à la température d'environ 70°C afin de réaliser l'émulsion.

b) Séparément on a ajouté 2 parties de polyvinylpyrrolidone réticulée et insolubilisée, 3 parties de sorbitol, et ensuite 0,05 parties d'acide sorbique et 0,1 partie de p-hydroxybenzoate de méthyl sodé.

c) On a d'autre part ajouté, à 30 parties de polyvinylpyrrolidone de masse moléculaire de 25 000, 33 parties d'eau déminéralisée et on a agité jusqu'à dissolution complète.

A moins de 70°C, on a ajouté sous agitation, 8,8 parties d'alginate de sodium et ensuite 7 parties de carboxyméthylcellulose insolubilisée. Une fois que cette préparation était bien homogène, on a maintenu la température à moins de 70°C et on y a ajouté, tout en agitant, les produits des étapes a et b. On a maintenu l'agitation pendant encore 15 minutes.

La pâte obtenue avait la composition suivante:

|  | Parties en poids, pour 100 parties en poids de pâte* |
|---|---|
| Polyvinylpyrrolidone réticulée et insolubilisée | 2,0 |
| Polyvinylpyrrolidone PM 25 000 | 30,0 |
| Carboxyméthylcellulose insolubilisée | 7,0 |
| Alginate de sodium | 8,80 |
| Sorbitol aqueux | 3,0 |
| Huile de paraffine | 3,20 |

|  | Parties en poids, pour 100 parties en poids de pâte* |
|---|---|
| Palmitostéarate de glycérol et de polyoxyéthylène glycol | 6,0 |
| Eau déminéralisée | 40,0 |

* sans conservateur

à laquelle s'ajoutaient encore 0,1 partie de p-hydroxybenzoate de méthyle sodé et 0,05 partie d'acide sorbique, comme conservateurs.

**Exemple 2**

a) On a ajouté 3,8 parties d'huile de paraffine à 7,1 parties de palmitostéarate de glycérol et de polyoxyéthylène glycol. On a porté le mélange à 70°C.

b) Séparément, à 2,3 parties de polyvinylpyrrolidone réticulée et insolubilisée, on a ajouté 3,6 parties de sorbitol, 0,05 partie d'acide sorbique et 0,1 partie de p-hydroxybenzoate de méthyle sodé.

c) On a ajouté, d'autre part, à 35,7 parties de polyvinylpyrrolidone PM = 25 000, sous agitation, 28,6 parties d'eau déminéralisée, et on a ensuite incorporé à cette préparation, sous agitation, 10,6 parties d'alginate de sodium et 8,3 parties de carboxyméthylcellulose insolubilisée.

On a ensuite ajouté à cette préparation le produit de l'étape b), en maintenant à une température inférieure à 70°C et sous agitation, on y a ajouté le produit de l'étape a). On a maintenu l'agitation pendant encore 15 minutes.

La pâte obtenue avait la composition suivante:

|  | Parties en poids, pour 100 parties en poids de pâte* |
|---|---|
| Polyvinylpyrrolidone réticulée et insolubilisée | 2,3 |
| Polyvinylpyrrolidone PM 25 000 | 35,7 |
| Carboxyméthylcellulose insolubilisée | 8,3 |
| Alginate de sodium | 10,6 |
| Sorbitol aqueux | 3,6 |
| Huile de paraffine | 3,8 |
| Palmitostéarate de glycérol et de polyoxyéthylène glycol | 7,1 |
| Eau déminéralisée | 28,6 |

* sans conservateur

à laquelle s'ajoutaient encore 0,1 partie de p-hydroxybenzoate de méthyle sodé et 0,05 partie d'acide sorbique.

**Exemple 3**

On a opéré comme indiqué de manière générale dans l'exemple 1 et on a obtenu une pâte ayant la composition ci-dessous, les proportions respectives des composants mis en œuvre étant celles mentionnées ci-après.

|  | Parties en poids, pour 100 parties en poids de pâte* |
|---|---|
| Polyvinylpyrrolidone réticulée et insolubilisée | 2,0 |
| Polyvinylpyrrolidone PM 25 000 | 30,0 |
| Carboxyméthylcellulose de sodium insolubilisée | 10,0 |
| Alginate de sodium | 10,0 |
| Sorbitol aqueux | 5,0 |
| Huile de paraffine | 5,0 |
| Palmitostéarate de glycérol et de polyoxyéthylène glycol | 8,0 |
| Eau déminéralisée | 30,0 |

* sans conservateur

à laquelle s'ajoutaient encore 0,05 partie d'acide sorbique et 0,1 partie de p-hydroxy-benzoate de méthyle sodé.

**Exemple 4 à 13**

On a opéré comme indiqué dans l'exemple 1, mais en faisant varier les quantités respectives des composants mis en œuvre. On a obtenu, abstraction faite de la présence éventuelle d'autres additifs, tels que des conservateurs, des pâtes selon l'invention qui étaient toutes appropriées aux usages mentionnés et dont les compositions étaient consignées dans le tableau I ci-après.

Pour chacune de ces pâtes, ainsi que pour celles des exemples 1 à 3, stéarate/(eau + huile + sorbitol) et PVP/eau ont été calculés; on a obtenu les valeurs figurant au tableau II ci-après.

Tableau I
Parties en poids pour 100 parties en poids de pâte

| Exemples | 4 | 5 | 6 | 7 | 8 | 9 | 10 | 11 | 12 | 13 |
|---|---|---|---|---|---|---|---|---|---|---|
| Polyvinylpyrrolidone réticulée et insolubilisée | 1,6 | 2,0 | 2,2 | 2,5 | 2,2 | 1,9 | 1,9 | 2,1 | 2,1 | 1,8 |
| Polyvinylpyrrolidone PM=25 000 | 29,9 | 29,9 | 33,4 | 36,5 | 40,0 | 23,9 | 35,5 | 37,6 | 31,8 | 29,4 |
| Carboxyméthylcellulose insolubilisée | 7,0 | 7,0 | 7,8 | 8,2 | 10,0 | 19,1 | 11,8 | 10,0 | 9,5 | 8,8 |
| Alginate de sodium | 2,8 | 8,8 | 9,8 | 10,7 | 10,3 | 19,1 | 11,8 | 10,0 | 11,0 | 10,3 |
| Sorbitol aqueux | 3,0 | 3,0 | 3,3 | 3,6 | 5,0 | 3,1 | 3,1 | 3,2 | 4,8 | 4,4 |
| Huile de Vaseline® | 3,3 | 3,3 | 3,3 | 3,9 | 4,5 | 3,1 | 3,1 | 3,2 | 4,8 | 4,4 |
| Palmitostéarate de glycérol et de polyoxyéthylène glycol | 6,0 | 6,0 | 6,6 | 7,3 | 8,0 | 5,9 | 9,2 | 8,8 | 4,2 | 11,5 |
| Eau déminéralisée | 46,4 | 40,0 | 33,4 | 27,3 | 20,0 | 23,9 | 23,6 | 25,1 | 31,8 | 29,4 |

Tableau II

| Exemples | 1 | 2 | 3 | 4 | 5 | 6 | 7 | 8 | 9 | 10 | 11 | 12 | 13 |
|---|---|---|---|---|---|---|---|---|---|---|---|---|---|
| Poudre/eau | 1,45 | 1,91 | 1,67 | 0,86 | 1,14 | 1,53 | 2,03 | 3,075 | 2,50 | 2,50 | 2,30 | 1,65 | 1,65 |
| Stéarate/(eau + huile + sorbitol aqueux) | 0,13 | 0,20 | 0,20 | 0,11 | 0,13 | 0,165 | 0,21 | 0,27 | 0,20 | 0,31 | 0,28 | 0,10 | 0,30 |
| PVP/eau | 0,75 | 1,25 | 1,00 | 0,64 | 0,75 | 1,00 | 1,34 | 2,00 | 1,00 | 1,50 | 1,50 | 1,00 | 1,00 |

Les pâtes obtenues avaient une consistance plus ou moins dure selon les cas, mais toutes étaient appropriées pour les usages, indiqués plus haut, auxquels elles sont plus spécialement destinées.

**Revendications pour les états contractants: BE, CH, DE, GB, IT, LI, LU, NL, SE**

1. Pâte de protection de la peau, caractérisée en ce qu'elle comprend fondamentalement:

a) au moins un hydrocolloïde et/ou un autre produit superabsorbant tiré d'hydrocolloïdes
b) de la carboxyméthylcellulose insolubilisée et
c) une polyvinylpyrrolidone ayant une masse moléculaire de l'ordre de 25 000 à 30 000,
d) de l'eau déminéralisée,
e) de l'huile et
f) un stéarate appropriées, ainsi que
g) de la polyvinylpyrrolidone réticulée et insolubilisée.

2. Pâte selon la revendication 1, caractérisée en ce qu'elle contient en outre du sorbitol et/ou du polyéthylène glycol.

3. Pâte selon la revendication 1, caractérisée en ce que les composants essentiels de la pâte sont dans les rapports et proportions ci-après, en poids/poids:
– poudre/eau 1 à 3 environ, de préférence 1,5 à 1,9;
– stéarate/(eau + huile + sorbitol et/ou polythyleneglycol) $\geq$ 0,10 de préférence 0,15 environ,
– polyvinylpyrrolidone eau 0,5 à 2 environ;
le terme de poudre désignant l'ensemble des composants pulvérulent.

4. Pâte selon l'une des revendication 1 à 3, caractérisée en ce que la polyvinylpyrrolidone réticulée et insolubilisée constitue au plus 3% en poids du poids de la pâte finie.

5. pâte selon l'une quelconque des revendications 1 à 4, caractérisée en ce que l'hydrocolloïde comprend un alginate alcalin.

6. Pâte selon l'une quelconque des revendications 1 à 5, caractérisée en ce que l'huile est une huile paraffinique.

7. Pâte selon l'une quelconque des revendications 1 à 6, caractérisée en ce que le stéarate est un stéarate d'alcools gras, notamment choisi parmi le palmitostéarate de glycérol et de polyoxyéthylène glycol, le palmitostéarate d'éthylène glycol et de polyéthylène glycol et le palmitostéarate de glycols associé à un phosphate d'alcool gras polyoxyéthyléné.

8. Application des pâtes selon l'une quelconque des revendications 1 à 7 pour la protection de la peau contre les écoulements corporels notamment contre les agressions des suintements des plaies et/ou des matières s'écoulant des stomies urinaires ou digestives.

9. Application des pâtes selon l'une quelconque des revendications 1 à 7, comme support et/ou véhicule neutre pour compositions pharmaceutiques pour administration percutanée prolongée d'un principe actif à diffusion transcutanée.

**Revendications pour l'état contractant: AT**

1. Procédé de préparation d'une pâte pour la protection de la peau caractérisé en ce qu'on mélange en chauffant à une température inférieure à 70°C
a) un hydrocolloïde et/ou un super absorbant tiré d'hydrocolloïdes
b) de la carboxyméthylcellulose insolubilisée
c) une polyvinylpyrrolidone de masse moléculaire de l'ordre de 25 000 à 30 000
d) de l'eau déminéralisée
e) de l'huile
f) un stéarate
ainsi que
g) de la polyvinylpyrrolidone réticulée et insolubilisée.

2. Procédé selon la revendication 1, caractérisé en ce qu'on ajoute à une solution de polyvinyl pyrrolidone dans de l'eau à moins de 70°C, de l'hydrocolloïde et de la carboxyméthylcellulose puis l'émulsion de l'huile et du stéarate dans l'eau et enfin la polyvinylpyrrolidone réticulée.

3. Procédé selon l'une des revendications précédentes caractérisé en ce qu'on ajoute en outre du sorbitol et/ou du polyéthylène glycol.

4. Procédé selon l'une des revendications précédentes caractérisé en ce que les proportions des mélanges sont en poids
– Composés pulvérulents/eau: 1 à 3 et de préférence 1,5 à 1,9
– stéarate/(eau + huile + sorbitol et/ou polyéthylèneglycol) 0,10 et de préférence 0,15.
– polyvinylpyrrolidone/eau: 0,5 à 2.

5. Procédé selon l'une des prévendications précédentes, caractérisé en ce que la polyvinylpyrrolidone réticulée et insolubilisée constitue au plus 3% en poids du poids de la pâte finie.

6. Procédé selon l'une quelconque des revendications 1 à 5, caractérisé en ce que l'hydrocolloïde comprend un alginate alcalin.

7. Procédé selon l'une quelconque des revendications 1 à 6, caractérisé en ce que l'huile est une huile paraffinique.

8. Procédé selon l'une quelconque des revendications 1 à 7, caractérisé en ce que le stéarate est un stéarate d'alcools gras, notamment choisi parmi le palmitostéarate de glycérol et de polyoxyéthylène glycol, le palmitostéarate d'éthylène glycol et de polyéthylène glycol et le palmitostéarate de glycols associé à un phosphate d'alcool gras polyoxyéthyléné.

**Patentansprüche für die Vertragsstaaten BE, CH, DE, GB, IT, LI, LU, NL, SE**

1. Paste für den Schutz der Haut, dadurch gekennzeichnet, dass sie im wesentlichen
a) ein Hydrokolloid und/oder ein von Hydrokolloiden abgeleitetes, in höchstem Masse absorbierendes Mittel,
b) unlöslich gemachte Carboxymethylcellulose,
c) ein Polyvinylpyrrolidon mit einem Molekulargewicht in der Grössenordnung von 25 000 bis 30 000,
d) entsalztes Wasser,
e) Öl,
f) ein Stearat und
g) vernetztes und unlöslich gemachtes Polyvinylpyrrolidon enthält.

2. Paste nach Anspruch 1, dadurch gekennzeichnet, dass sie ausserdem Sorbit und/oder Polyethylenglycol enthält.

3. Paste nach Anspruch 1, dadurch gekennzeichnet, dass die wesentlichen Bestandteile der Paste in den folgenden Gewichtsverhältnissen vorliegen:
Pulver/Wasser: 1 bis 3, vorzugsweise 1,5 bis 1,9,
Stearat (Wasser + Öl + Sorbit und/oder Polyethylenglycol): $\geq$ 0,10, vorzugsweise 0,15,
Polyvinylpyrrolidon/Wasser: 0,5 bis 2,
wobei der Ausdruck «Pulver» die Gesamtheit der pulverförmigen Bestandteile umfasst.

4. Paste nach einem der Ansprüche 1 bis 3, dadurch gekennzeichnet, dass das vernetzte und unlöslich gemachte Polyvinylpyrrolidon höchstens 3 Gew.% der fertigen Paste ausmacht.

5. Paste nach irgendeinem der Ansprüche 1 bis 4, dadurch gekennzeichnet, dass das Hydrokolloid ein Alkalialginat umfasst.

6. Paste nach irgendeinem der Ansprüche 1 bis 5, dadurch gekennzeichnet, dass das Öl ein Paraffinöl ist.

7. Paste nach irgendeinem der Ansprüche 1 bis 6, dadurch gekennzeichnet, dass das Stearat ein Stearat von Fettalkoholen ist und insbesondere aus Glycerinpalmitostearat und Polyoxyethylenglycol, Ethylenglycolpalmitostearat und Polyethylenglycol sowie einem mit einem polyethoxylierten Fettalkoholphosphat assoziierten Palmitostearat von Glycolen ausgewählt ist.

8. Anwendung der Pasten nach irgendeinem der Ansprüche 1 bis 7 zum Schutz der Haut gegen Körperausflüsse, insbesondere gegen das Nässen von Wunden und/oder Stoffe, die aus den Urintrakten oder Verdauungstrakten ausfliessen.

9. Anwendung der Pasten nach irgendeinem der Ansprüche 1 bis 7 als Träger und/oder neutrales Vehikel für pharmazeutische Zusammensetzungen für die verlängerte percutane Anwendung eines Wirkstoffes durch transcutane Diffusion.

**Patentansprüche für den Vertragsstaat MT**

1. Verfahren zur Herstellung einer Paste für den Schutz der Haut, dadurch gekennzeichnet, dass man unter Erwärmen auf eine Temperatur unter 70°C

a) ein Hydrokolloid und/oder ein von Hydrokolloiden abgeleitetes in höchstem Masse absorbierendes Mittel,

b) unlöslich gemachte Carboxymethylcellulose,

c) ein Polyvinylpyrrolidon mit einem Molekulargewicht in der Grössenordnung von 25 000 bis 30 000,

d) entsalztes Wasser,

e) Öl,

f) ein Stearat und

g) vernetztes und unlöslich gemachtes Polyvinylpyrrolidon mischt.

2. Verfahren nach Anspruch 1, dadurch gekennzeichnet, dass man zu einer Lösung von Polyvinylpyrrolidon in Wasser von wenigstens 70°C das Hydrokolloid und die Carboxymethylcellulose und dann die Emulsion von Öl und Stearat in Wasser und schliesslich das vernetzte Polyvinylpyrrolidon gibt.

3. Verfahren nach einem der vorhergehenden Ansprüche, dadurch gekennzeichnet, dass man ausserdem Sorbit und/oder Polyethylenglycol zugibt.

4. Verfahren nach einem der vorhergehenden Ansprüche, dadurch gekennzeichnet, dass die Gewichtsverhältnisse der Gemische die folgenden sind:

pulverförmige Verbindungen/Wasser: 1 bis 3, vorzugsweise 1,5–1,9,

Stearat (Wasser + Öl + Sorbit und/oder Polyethylenglycol): 0,10, vorzugsweise 0,15,

Polyvinylpyrrolidon/Wasser: 0,5 bis 2.

5. Verfahren nach einem der vorhergehenden Ansprüche, dadurch gekennzeichnet, dass das vernetzte und unlöslich gemachte Polyvinylpyrrolidon höchstens 3 Gew.% der fertigen Paste ausmacht.

6. Verfahren nach irgendeinem der Ansprüche 1 bis 5, dadurch gekennzeichnet, dass das Hydrokolloid ein Alkalialginat umfasst.

7. Verfahren nach irgendeinem der Ansprüche 1 bis 6, dadurch gekennzeichnet, dass das Öl ein Paraffinöl ist.

8. Verfahren nach irgendeinem der Ansprüche 1 bis 7, dadurch gekennzeichnet, dass das Stearat ein Stearat von Fettalkoholen ist und insbesondere aus Glycerinpalmitostearat und Polyoxyethylenglycol, Ethylenglycolpalmitostearat und Polyethylenglycol sowie einem mit einem polyethoxylierten Fettalkoholphosphat assoziiertem Palmitostearat von Glycolen ausgewählt ist.

**Claims for the Contracting States: BE, CH, DE, GB, IT, LI, LU, NL, SE**

1. Paste for protecting the skin, characterized in that it is essentially comprises:

a) at least one hydrocolloid and/or another superabsorbent product obtained from hydrocolloids,

b) insolubilized carboxymethylcellulose, and

c) a polyvinylpyrrolidone having a molecular mass of the order of 25,000 to 30,000,

d) demineralized water,

e) a suitable oil and

f) a suitable stearate, and also

g) crosslinked and insolubilized polyvinylpyrrolidone.

2. Paste according to Claim 1, characterized in that it contains in addition sorbitol and/or polyethylene glycol.

3. Paste according to Claim 1, characterized in that the essential components of the paste are in the weight/weight ratios and proportions below:

powder/water about 1 to 3, preferably 1.5 to 1.9;

stearate/(water plus oil plus sorbitol and/or polyethylene glycol) $\geq 0.10$, preferably about 0.15, polyvinylpyrrolidone/water about 0.5 to 2;

The term powder denoting the combination of powdered components.

4. Paste according to one of Claims 1 to 3, characterized in that the crosslinked and insolubilized polyvinylpyrrolidone constitutes at most 3% by weight of the weight of the finished paste.

5. Paste according to any one of Claims 1 to 4, characterized in that the hydrocolloid comprises an alkali metal alginate.

6. Paste according to any one of Claims 1 to 5, characterized in that the oil is a paraffinic oil.

7. Paste according to any one of Claims 1 to 6, characterized in that the stearate is a stearate of fatty alcohols chosen, in particular, from glycerol and polyoxyethylene glycol palmitate/stearate, ethylene glycol and polyethylene glycol palmitate/stearate, and glycol palmitate/stearate in combination with a phosphate of a polyoxyethylenated fatty alcohol.

8. Application of the paste according to any one of Claims 1 to 7 for protecting the skin against discharges of body fluids, in particular against attack by exudations from wounds and/or by substances discharging from urinary or digestive stomas.

9. Application of the paste according to any one of Claims 1 to 7 as a carrier and/or neutral vehicle for pharmaceutical compositions for prolonged percutaneous administration of an active principle having the property of transcutaneous diffusion.

**1. Claims for the Contracting State: AT**

1. Process for preparing a paste for the protection of the skin, characterized in that the following are mixed while being heated to a temperature below 70°C:

a) a hydrocolloid and/or a superabsorbent obtained from hydrocolloids,

b) insolubilized carboxymethylcellulose,

c) a polyvinylpyrrolidone of molecular mass of the order of 25,000 to 30,000,

d) demineralized water,

e) oil,

f) stearate, and also

g) crosslinked and insolubilized polyvinylpyrrolidone.

2. Process according to Claim 1, characterized in that hydrocolloid and carboxymethylcellulose are added to a solution of polyvinylpyrrolidone in water at less than 70°C, followed by an emulsion of the oil and the stearate in water and finally the crosslinked polyvinylpyrrolidone.

3. Process according to one of the preceding claims, characterized in that sorbitol and/or polyethylene glycol are also added.

4. Process according to one of the preceding claims, characterized in that the proportions by weight of the mixtures are:

powdered compounds/water: 1 to 3 and preferably 1.5 to 1.9,

stearate/(water plus oil plus sorbitol and/or polyethylene glycol): 0.10 and preferably 0.15, polyvinylpyrrolidone/water: 0.5 to 2.

5. Process according to one of the preceding claims, characterized in that the crosslinked and insolubilized polyvinylpyrrolidone constitutes at most 3% by weight of the weight of the finished paste.

6. Process according to any one of Claims 1 to 5, characterized in that the hydrocolloid comprises an alkali metal alginate.

7. Process according to any one of Claims 1 to 6, characterized in that the oil is a paraffinic oil.

8. Paste according to any one of Claims 1 to 7, characterized in that the stearate is a stearate of fatty alcohols chosen, in particular, from glycerol and polyoxyethylene glycol palmitate/stearate, ethylene glycol and polyethylene glycol plamitate/stearate, and glycol palmitate/stearate in combination with a phosphate of a polyoxyethylenated fatty alcohol.